# EUROPÄISCHE PATENTANMELDUNG

(11) **EP 4 628 122 A1**
(43) Veröffentlichungstag der Anmeldung: **08.10.2025**
(21) Anmeldenummer: 25166682.2
(22) Anmeldetag: 27.03.2025
(51) Int. Cl.: A61M 1/36, A61M 60/113, A61M 60/38, A61M 60/50, A61M 60/531, A61M 60/546

(54) **ANORDNUNG ZUR ETABLIERUNG EINES EXTRAKORPORALEN BLUTKREISLAUFES MIT EINEM SCHLAUCHLEITUNGSSYSTEM**

(30) Priorität: 03.04.2024 DE 102024109290
(71) Anmelder: ResuSciTec GmbH, 79108 Freiburg (DE)
(72) Erfinder: Grudke, Jürgen, 79108 Freiburg (DE); Heß, Christian, 79108 Freiburg (DE); Hoff, Christian, 79108 Freiburg (DE); Benk, Christoph, 79108 Freiburg (DE)
(74) Vertreter: Rösler, Uwe

(57) **Zusammenfassung**

Die Erfindung bezieht sich auf eine Anordnung zur Etablierung eines extrakorporalen Blutkreislaufes mit einem Schlauchleitungssystem, das über einen Ein- und Auslass verfügt, die geeignet ausgebildet sind an einen Blutkreislauf eines Individuums applizierbar zu sein, und längs dem zwei Fluidpumpen angeordnet sind, von denen eine erste Fluidpumpe einen kontinuierlichen Fluidstrom innerhalb des Schlauchleitungssystems zu erzeugen vermag und von denen eine zweite Fluidpumpe einen pulsatilen Fluidstrom zu erzeugen vermag , wobei zwischen beiden Fluidpumpen wenigstens eine therapeutisch wirksame Komponente längs des Schlauchleitungssystems angeordnet ist.

Die Erfindung zeichnet sich dadurch aus, dass die zweite Fluidpumpe mit einer Kontrolleinheit verbunden ist, die wenigstens einen Betriebsparameter der zweiten Fluidpumpe wenigstens in Abhängigkeit einer vorgebbaren Drehzahl, mit der die erste Fluidpumpe betreibbar ist, kontrolliert.

## Beschreibung

### Technisches Gebiet

Die Erfindung bezieht sich auf eine Anordnung zur Etablierung eines extrakorporalen Blutkreislaufes mit einem Schlauchleitungssystem, das über einen Ein- und Auslass verfügt, die geeignet ausgebildet sind an einen Blutkreislauf eines Individuums applizierbar zu sein, und längs dem zwei Fluidpumpen angeordnet sind, von denen eine erste Fluidpumpe einen kontinuierlichen Fluidstrom innerhalb des Schlauchleitungssystems zu erzeugen vermag und vorzugsweise stromab des Einlasses angeordnet ist und von denen eine zweite Fluidpumpe einen pulsatilen Fluidstrom zu erzeugen vermag und vorzugsweise stromab zur ersten Fluidpumpe angeordnet ist, wobei zwischen beiden Fluidpumpen wenigstens eine therapeutisch wirksame Komponente längs des Schlauchleitungssystems angeordnet ist.

### Stand der Technik

Extrakorporale Fluidkreisläufe dienen zur Entnahme und Wiederrückführung von körpereigenen Flüssigkeiten aus einem sowie in ein Lebewesen, vorzugsweise zum Zwecke einer therapeutischen Manipulation bzw. Behandlung der körpereigenen Flüssigkeit, vorzugsweise von Blut, bspw. mittels Filtration, Oxygenierung, Stoffzugabe etc. und/oder zur Etablierung eines Fluid-Bypasses zu einem bestimmten körpereigenen Flüssigkeits-Kreislaufabschnittes.

Die Druckschrift DE 36 90 585 T1 beschreibt eine externe, pulsierende Herzunterstützungs-Vorrichtung mit einem extrakorporalen Blutkreislauf, über den mittels einer an der Oberschenkelvene applizierbaren Kanüle venöses Blut aus einem Patienten durch Unterstützung einer, eine kontinuierliche Sogwirkung erzeugenden Rollenpumpe entnommen wird. Längs des extrakorporalen Blutkreislaufes ist stromab zur Rollenpumpe ein Oxygenator angeordnet, der das venöse Blut mit Sauerstoff anreichert. Dem Oxygenator längs des extrakorporalen Blutkreislaufes nachfolgend ist eine pulsierend betreibbare Förderpumpe angeordnet, durch die der ansonsten kontinuierliche extrakorporale Blutstrom mit einer pulsierenden Druckamplitude derart moduliert wird, so dass die Druckpulse in Abhängigkeit der absteigenden T-Zacke des Herzens des Patienten beginnen. Zwischen der pulsierend arbeitenden Pumpe und der Rückführung des extrakorporalen Blutkreislaufes in den Patienten ist zudem ein Blutfilter angeordnet.

Die Druckschrift DE 10 2013 012 433 A1 beschreibt eine Anordnung zur Etablierung eines extrakorporalen Blutkreislaufes mit einem Schlauchleitungssystem, längs dem wenigstens eine Pumpe angeordnet ist, die derart ausgebildet und ansteuerbar ist, dass eine wellenartig an- und abschwellende Pumpenleistung für eine pulsatile Strömung längs des Schlauchleitungssystems erzeugbar ist. Alternativ kann dies auch mit zwei Pumpen realisiert werden, wovon eine Pumpe eine konstante Grundlast und die andere Pumpe eine an- und abschwellenden Pumpenleistung erzeugt.

### Darstellung der Erfindung

Der Erfindung liegt die Aufgabe zugrunde die Betriebssicherheit eines vorstehend erläuterten extrakorporalen Blutkreislaufes, in dem zwei Pumpen jeweils zur Etablierung eines kontinuierlichen sowie eines pulsatilen Blutflusses integriert sind, sowohl im Hinblick auf die Patientensicherheit und einer möglichst schonenden und geringen Belastung des Patienten im Rahmen einer notwendigen Etablierung des extrakorporalen Blutkreislaufes als auch im Hinblick auf die Funktionssicherheit der wenigstens einen therapeutisch wirksamen Komponente, die längs des extrakorporalen Blutkreislaufes angeordnet ist, zu verbessern.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie im Weiteren unter Bezugnahme auf ein illustriertes Ausführungsbeispiel zu entnehmen.

Eine lösungsgemäße Anordnung zur Etablierung eines extrakorporalen Blutkreislaufes mit einem Schlauchleitungssystem, das über einen Ein- und Auslass verfügt, die geeignet ausgebildet sind an einen Blutkreislauf eines Individuums applizierbar zu sein, und längs dem zwei Fluidpumpen angeordnet sind, von denen eine erste Fluidpumpe einen kontinuierlichen Fluidstrom innerhalb des Schlauchleitungssystems zu erzeugen vermag und vorzugsweise stromab des Einlasses angeordnet ist, und von denen eine zweite Fluidpumpe einen pulsatilen Fluidstrom zu erzeugen vermag und vorzugsweise stromab zur ersten Fluidpumpe angeordnet ist, wobei zwischen beiden Fluidpumpen wenigstens eine therapeutisch wirksame Komponente längs des Schlauchleitungssystems angeordnet ist, zeichnet sich dadurch aus, dass die zweite Fluidpumpe mit einer Kontrolleinheit verbunden ist, die wenigstens einen Betriebsparameter der zweiten Fluidpumpe wenigstens in Abhängigkeit einer vorgebbaren Drehzahl, mit der die erste Fluidpumpe betreibbar ist, kontrolliert.

Durch die lösungsgemäße Betriebsabhängigkeit der zweiten, pulsatil arbeitenden Fluidpumpe zumindest von der Betriebsdrehzahl der ersten kontinuierlich arbeitenden Fluidpumpe kann durch die Wahl einer der Betriebsabhängigkeit zugrunde liegenden Regelungs- bzw. Steuerungspolitik ein sicherer Pumpenbetrieb automatisiert erfolgen, der sowohl auf sich möglicherweise ändernde Betriebszustände der wenigstens einen im Schlauchleitungssystem integrierten therapeutischen Komponente sowie auch und insbesondere auf sich möglicherweise ändernde patientenspezifische physiologische Parameter reagieren kann. So ermöglicht vorzugsweise die lösungsgemäße Anordnung die wenigstens zwei Pumpen innerhalb des extrakorporalen Schlauchleitungssystems sowohl vorlastabhängig, also in Abhängigkeit eines von Seiten des Patienten abfließenden bzw. herrührenden Blutvolumenstroms, sowie auch nachlastsensitiv, d.h. in Abhängigkeit eines in den Patienten maximal rückfließenden Blutvolumenstroms, sicher zu betreiben.

Vorzugsweise regelt bzw. beeinflusst die Kontrolleinheit dabei wenigstens einen der nachfolgenden Betriebsparameter der zweiten Fluidpumpe: Drehzahl, Systolen-Dauer des durch die zweite Fluidpumpe erzeugbaren pulsatilen Fluidstroms, Frequenz und/oder Amplitude des durch die zweite Fluidpumpe erzeugbaren pulsatilen Fluidstroms.

Als Grundlage für die Regelung bzw. Steuerung dient zumindest die Drehzahl der ersten Fluidpumpe, die vorzugsweise über ein an der Kontrolleinheit vorgesehenes oder mit der Kontrolleinheit verbundenes Eingabemittel manuell oder akustisch durch einen Bediener eingebbar ist. Die Kontrolleinheit sorgt im Weiteren dafür, dass die zweite Fluidpumpe mit einer gegenüber der vorgegebenen Drehzahl, mit der die erste Fluidpumpe betreibbar ist, kleineren Drehzahl betrieben wird, um auf diese Weise sicherzustellen, dass der durch die erste Fluidpumpe innerhalb des Schlauchleitungssystems etablierte Strömungsdruck stets größer ist als jener, der dem Fluidstrom durch die pulsierend arbeitende zweite Fluidpumpe in aufmodulierender Weise hinzugefügt wird. Die zwischen beiden Fluidpumpen einstellbare Drehzahldifferenz, auch als Safety Margin bezeichnet, stellt sicher, dass sich längs des Schlauchleitungssystems zwischen beiden Fluidpumpen, zwischen denen die wenigstens eine therapeutisch wirksame Komponente, vorzugsweise in Form eines Oxygenators zur Sauerstoffanreicherung des venösen Blutstromes angeordnet ist, ein positiver Druckabfall einstellt. Ein negativer Druckabfall würde bspw. bei einem als Membran-Oxygenator ausgebildeten Oxygenator zu einem Luftübertritt über die Gasaustauschmembran sowie zu Kavitationserscheinungen führen, dies gilt es sicher auszuschließen.

In einer weiteren bevorzugten Ausführungsform ist längs des Schlauchleitungssystems wenigstens ein Sensor der nachfolgenden Art angeordnet: Drucksensor, Strömungsfluss-Sensor, Blasensensor. Die von den Sensoren erzeugbaren Sensorsignale sind vorzugsweise allesamt an die Kontrolleinheit übertragbar und dienen als weitere Kontrollgrößen im Sinne von Steuer- bzw. Regelgrößen, zur Beeinflussung wenigstens eines Betriebsparameters der zweiten Fluidpumpe.

Die Kontrolleinheit ist in vorteilhafter Weise als Chip-basierte Numerikeinheit ausgebildet und vermag die vorgegebene Kontrollpolitik im Rahmen eines Kontrollalgorithmus umzusetzen, mit dem der wenigstens eine Betriebsparameter der zweiten Fluidpumpe beeinflusst wird. So vermag die Kontrolleinheit die zweite Fluidpumpe in Abhängigkeit von wenigstens der Drehzahl der ersten Fluidpumpe automatisch zu starten, zu regeln und wieder zu deaktivieren bzw. zu stoppen.

Vorzugsweise werden im Rahmen des Kontrollalgorithmus bedarfsweise, d.h. durch manuelle Vorgabe oder in Abhängigkeit von sensorisch erfassten und bestimmten Betriebszuständen wenigstens eine der nachfolgenden Kontrollregeln umgesetzt:
Bei Erhöhung der Drehzahl der ersten Fluidpumpe wird zeitlich versetzt die Drehzahl der zweiten Fluidpumpe derart nachgeregelt, so dass sich der vorgegebene Unterschied (Safety Margin), zwischen beiden Drehzahlen nicht verkleinert.

Im Falle der Reduzierung der Drehzahl der ersten Fluidpumpe wird zeitlich zuerst die Drehzahl der zweiten Fluidpumpe reduziert und nachfolgend die Drehzahl der ersten Fluidpumpe vermindert.

Bei einer vorgegebenen Signalhöhe und/oder Signalqualität eines Sensorsignals wenigstens eines der längs des extrakorporalen Schlauchleitungssystems angeordneten Sensoren wird die Drehzahl der zweiten Fluidpumpe reduziert. Sollte trotz Vorhandensein wenigstens eines Sensors längs des Schlauchleitungssystems kein Sensorsignal vorliegen, so wird die zweite Fluidpumpe deaktiviert.

In einem weiteren Ausführungsbeispiel ist die erste Fluidpumpe derart ausgewählt, so dass sie in zwei Betriebsmodi betreibbar ist, einem Standard-Modus, in dem die erste Fluidpumpe wie vorstehend erläutert, einen kontinuierlichen Fluidstrom innerhalb des Schlauchleitungssystems zu erzeugen vermag, und einen zweiten Betriebsmodus, in dem die erste Fluidpumpe eine dem kontinuierlichen Fluidstrom zusätzliche Pulsatilität aufzuprägen vermag. Der zweite Betriebsmodus ist vorzugsweise durch die Kontrolleinheit aktivierbar und deaktivierbar. Insbesondere durch die vermittels der zweiten Fluidpumpe erzeugbare Pulsatilität kann die durch die erste Fluidpumpe hervorgerufene Pulsatilität verstärkt werden. Es sind jedoch auch sich zeitlich überlappende Pulsatilitäten innerhalb des Strömungsflusses mittels zweier pulsatil arbeitender Fluidpumpen realisierbar. Hierzu gilt es beide Fluidpumpen mit einer asynchronen pulsatilen Ansteuerung untereinander zu betreiben.

### Kurze Beschreibung der Erfindung

Die Erfindung wird nachstehend ohne Beschränkung des allgemeinen Erfindungsgedankens anhand eines bevorzugten Ausführungsbeispiels unter Bezugnahme auf die Zeichnungen exemplarisch beschrieben. Es zeigen:
- Fig. 1: schematisierte Darstellung eines extrakorporalen Blutkreislaufes mit zwei Fluidpumpen, sowie
- Fig. 2: Diagramm zur Darstellung eines Drehzahlvergleiches zwischen der ersten und zweiten Fluidpumpe.

### Wege zur Ausführung der Erfindung, gewerbliche Verwendbarkeit

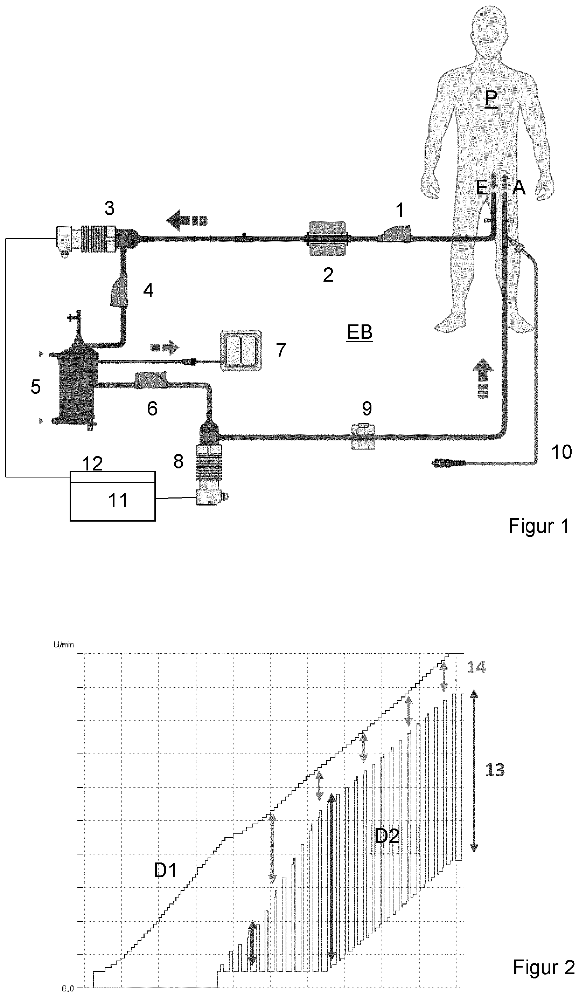

Figur 1 zeigt schematisiert einen extrakorporalen Blutkreislauf EB mit einem Einlass E, der in der Oberschenkelvene einer Person P appliziert ist sowie einem Auslass A, der in der Oberschenkelarterie der Person P appliziert ist. In Strömungsrichtung dem Einlass E nachfolgend sind die folgenden mit Bezugszeichen versehenen Komponenten längs des extrakorporalen Schlauchleitungssystems, der dem extrakorporalen Blutkreislauf EB entspricht, angeordnet: Drucksensor 1 zur Erfassung des venösen Blutdruckes p1, optischer Sensor 2 zur venösen Blutgasanalyse sowie zur Temperaturmessung, erste kontinuierlich arbeitende Fluidpumpe 3, weiterer Drucksensor 4, Oxygenator 5 zur Sauerstoffanreicherung des aus der Person P entnommenen venösen Blutes, weiterer Drucksensor 6, pulsatil arbeitende zweite Fluidpumpe 8, Blutfluss und Blasensensor 9, Bypass-Leitung zur arteriellen Blutdruckmessung 10. Ferner ist eine mit der zweiten Fluidpumpe 8 verbundene Kontrolleinheit 11 vorgesehen, die mit einem Eingabemittel 12 verbunden ist, über das die Betriebsdrehzahl für die erste Fluidpumpe 3 eingebbar ist. Optional ist der Oxygenator 5 darüber hinaus mit einer weiteren BlutgasanalyseEinheit 7 fluidisch verbunden.

Über das vorzugsweise taktil bedienbare Eingabemittel 12 gibt ein Anwender die Solldrehzahl für den Betrieb der ersten Fluidpumpe 3 vor. Die Drehzahl der zweiten Fluidpumpe 8 wird von der Kontrolleinheit 11 automatisch in Abhängigkeit der vorgegebenen Solldrehzahl der ersten Fluidpumpe 3 sowie optional unter Zugrundelegung weiterer Parameter, wie beispielsweise Sensorsignale der vorstehend erläuterten Sensoren 1, 2, 4, 6, 9 eingestellt.

Zudem kann optional von Seiten eines Anwenders über das Eingabemittel 12 eine Drehzahldifferenz (Safety Margin) zwischen beiden Drehzahlen vorgegeben oder von Seiten der Kontrolleinheit 11 in Abhängigkeit von sensoriell erfassbaren Betriebs- bzw. Rahmenbedingungen etabliert werden. In erster Linie soll die Safety Margin patientenbedingte instabile Perfusionsverhältnisse zu verhindern helfen.

Die vorzugsweise in Form einer Chip-basierten Numerikeinheit ausgebildete Kontrolleinheit 11 nutzt auf der Grundlage wenigstens eines vorgegebenen Kontrollalgorithmus zur Regelung der zweiten Fluidpumpe 8 neben der vorgegebenen Solldrehzahl, mit der die erste Fluidpumpe 3 betrieben wird, optional einzelne oder alle Sensorsignale, der im extrakorporalen Blutkreislauf in Figur 1 enthaltenen Sensoren. In Abhängigkeit des jeweils sensoriell überwachten und erfassten Istzustandes des mit der Person P verbundenen extrakorporalen Blutkreislaufes und/oder der vorgegebenen Solldrehzahl zum Betrieb der ersten Fluidpumpe 3 und/oder der vorgegebenen Drehzahldifferenz (Safety Margin) können neben der Drehzahl der zweiten Fluidpumpe 8 weitere Pumpenparameter der zweiten Fluidpumpe 8 geregelt bzw. beeinflusst werden, wie beispielsweise die Systolen-Dauer des durch die zweite Fluidpumpe 8 erzeugbaren pulsatilen Fluidstroms, die Frequenz und/oder die Amplitude des durch die zweite Fluidpumpe 8 erzeugbaren pulsatilen Fluidstroms.

Die dem Kontrollalgorithmus zugrunde liegende Regelungslogik berücksichtigt in allen denkbaren Regelungssituationen an aller erster Stelle das Patientenwohl sowie die zuverlässige Funktionalität sämtlicher im extrakorporalen Blutkreislauf enthaltenen Systemkomponenten.

Der Figur 2 ist eine Diagrammdarstellung zu entnehmen, die die Drehzahl D1 der ersten Fluidpumpe 3 sowie die Drehzahl D2 der zweiten Fluidpumpe 8 zeigt. Längs der Abszisse der in Figur 2 dargestellten Diagrammdarstellung ist die Zeitachse, längs der Ordinate ist die Drehzahl in Umdrehungen pro Minute angegeben. Bei niedrigen Drehzahlen D1 der ersten Fluidpumpe 1 sind die Amplituden 13, mit denen die Drehzahl D2 der zweiten Fluidpumpe variieren, reduziert und somit klein. Die Drehzahl D2 ist stets kleiner gewählt als die Drehzahl D1. Die Drehzahldifferenz D1 - D2 ist als Safety Margin 14 in Figur 2 illustriert. Bei geringeren Drehzahlen ist die Safety Margin 14 vorzugsweise größer als bei höheren Drehzahlen.

Werden Instabilitäten beim Perfusionsvorgang des Patienten P detektiert, beispielsweise mit Hilfe der längs des extrakorporalen Schlauchsystems integrierten Sensoren, so wird die Drehzahldifferenz 14, d.h. die Safety Margin, vorzugsweise automatisch erhöht. Fehlen andererseits Sensorwerte oder wird ein unsicherer Systemzustand oder Perfusionszustand erkannt, so wird die zweite Fluidpumpe 8 mittels der Kontrolleinheit 11 automatisch deaktiviert.

Wird hingegen die Drehzahl D1 der ersten Fluidpumpe 1 durch den Anwender erhöht, so sorgt die Kontrolleinheit 11 automatisch dafür, dass zur Einhaltung der Safety Margin 14 immer zuerst die Drehzahl D1 der ersten Fluidpumpe 3 verändert wird und zeitlich verzögert die Drehzahl D2 der zweiten Fluidpumpe 8. Wird hingegen die Drehzahl D1 der ersten Fluidpumpe 3 reduziert, so sorgt die Kontrolleinheit 11 dafür, dass zuerst die Drehzahl D2 der zweiten Fluidpumpe 8 reduziert wird.

Da sich die zweite Fluidpumpe 8 stromab längs des extrakorporalen Schlauchleitungssystems zum Oxygenator 5 befindet, vermag die zweite, pulsatil betriebene Fluidpumpe 8 selbst bei niedrigen Drehzahlen D2 einen hohen Energieeintrag in den Blutkreislauf des Patienten P ohne Dämpfung durch den Oxygenator 5 zu erzielen. Um eine negative Druckausbildung stromab, d.h. auf der Auslassseite des Oxygenators 5 zu verhindern, berücksichtigt die Kontrolleinheit 11 die Sensorsignale sämtlicher im extrakorporalen Schlauchleitungssystems angebrachter Drucksensoren 1, 4 und 6, so dass bei einem erfassten Druckabfall stromab des Oxygenators 8 unterhalb eines als kritisch definierten Schwellwerts die Kontrolleinheit 11 die Drehzahl D2 der zweiten Fluidpumpe 8 automatisch auf Null Umdrehungen pro Minute setzt, bis der Druck wieder über einen vorgegebenen Schwellwert zu liegen kommt. Auf diese Weise werden ein Luftübertritt über die Gasaustauschmembran des Oxygenators 5 und Kavitation verhindert.

### Bezugszeichenliste

- P: Patient
- E: Einlass
- A: Auslass
- EB: extrakorporaler Blutkreislauf
- 1: erster Drucksensor p1
- 2: optischer Sensor zur venösen Blutgasanalyse und Temperaturerfassung
- 3: erste Fluidpumpe
- 4: zweiter Drucksensor p2
- 5: Oxygenator
- 6: dritter Drucksensor p3
- 7: Blutgasanalyseeinheit
- 8: zweite Fluidpumpe
- 9: Strömungsfluss- und Blasensensor
- 10: arterieller Drucksensor
- 11: Kontrolleinheit
- 12: Eingabemittel
- 13: Amplitude
- 14: Drehzahldifferenz, Safety Margin

## Patentansprüche

1. Anordnung zur Etablierung eines extrakorporalen Blutkreislaufes (EB) mit einem Schlauchleitungssystem, das über einen Ein- und Auslass (E, A) verfügt, die geeignet ausgebildet sind an einen Blutkreislauf eines Individuums (P) applizierbar zu sein, und längs dem zwei Fluidpumpen (3, 8) angeordnet sind, von denen eine erste Fluidpumpe (3) einen kontinuierlichen Fluidstrom innerhalb des Schlauchleitungssystems zu erzeugen vermag und von denen eine zweite Fluidpumpe (8) einen pulsatilen Fluidstrom zu erzeugen vermag, wobei zwischen beiden Fluidpumpen (3, 8) wenigstens eine therapeutisch wirksame Komponente längs des Schlauchleitungssystems angeordnet ist,
**dadurch gekennzeichnet, dass** die zweite Fluidpumpe (8) mit einer Kontrolleinheit (11) verbunden ist, die wenigstens einen Betriebsparameter der zweiten Fluidpumpe (8) wenigstens in Abhängigkeit einer vorgebbaren Drehzahl (D1), mit der die erste Fluidpumpe (3) betreibbar ist, regelt.

2. Anordnung nach Anspruch 1,
**dadurch gekennzeichnet, dass** der wenigstens eine Betriebsparameter der zweiten Fluidpumpe (8) wenigstens einer der nachfolgenden Betriebsparameter ist: Drehzahl (D2), Systolen-Dauer des durch die zweite Fluidpumpe erzeugbaren pulsatilen Fluidstroms, Frequenz und/oder Amplitude (13) des durch die zweite Fluidpumpe erzeugbaren pulsatilen Fluidstroms.

3. Anordnung nach Anspruch 1 oder 2,
**dadurch gekennzeichnet, dass** die Kontrolleinheit (11) ein Eingabemittel (12) vorsieht, über die die Drehzahl (D1) der ersten Fluidpumpe (3) vorgebbar ist.

4. Anordnung nach Anspruch 3,
**dadurch gekennzeichnet, dass** die zweite Fluidpumpe (8) mit einer Drehzahl (D2) betreibbar ist, die kleiner als die Drehzahl (D1) der ersten Fluidpumpe (3) ist, und dass über das Eingabemittel (12) oder ein weiteres Eingabemittel ein Unterschied (14) zwischen beiden Drehzahlen (D1, D2) eingebbar ist, der der Kontrolleinheit (11) zur Kontrolle des wenigstens einen Betriebsparameters der zweiten Fluidpumpe (8) vorgebbar ist.

5. Anordnung nach einem der Ansprüche 1 bis 4,
**dadurch gekennzeichnet, dass** längs des Schlauchleitungssystems wenigstens ein Sensor der nachfolgenden Art angeordnet ist: Drucksensor, Strömungsflusssensor, Blasensensor,
und dass der wenigstens eine Sensor ein Sensorsignal erzeugt, das an die Kontrolleinheit (11) übertragbar ist.

6. Anordnung nach Anspruch 4 oder 5,
**dadurch gekennzeichnet, dass** die Kontrolleinheit (11) eine Chip-basierte Numerikeinheit aufweist, die auf Grundlage eines Kontrollalgorithmus die Regelung des wenigstens einen Betriebsparameters der zweiten Fluidpumpe (8) vornimmt, und dass sich der Kontrollalgorithmus durch wenigstens eine der folgenden Kontrollregeln auszeichnet:
- Bei Erhöhung der Drehzahl (D1) der ersten Fluidpumpe (3) wird zeitlich versetzt die Drehzahl (D2) der zweiten Fluidpumpe (8) derart nachgeregelt, so dass sich der vorgegebene Unterschied (14) zwischen beiden Drehzahlen nicht verkleinert,
- Bei Reduzierung der Drehzahl (D1) der ersten Fluidpumpe (3) wird zeitlich zuerst die Drehzahl (D2) der zweiten Fluidpumpe (8) reduziert und nachfolgend die Drehzahl (D1) der ersten Fluidpumpe (3),
- Bei einer vorgebbaren Signalhöhe und/oder Signalqualität des Sensorsignals des wenigstens einen Sensors wird die Drehzahl (D2) der zweiten Fluidpumpe (8) verändert, vorzugsweise reduziert,
- Bei Fehlen eines Sensorsignals wird die zweite Fluidpumpe (8) deaktiviert.

7. Anordnung nach einem der Ansprüche 1 bis 6,
**dadurch gekennzeichnet, dass** zwischen dem Einlass (E) und der ersten Fluidpumpe (3) ein erster Drucksensor (1) und zwischen diesem und der ersten Fluidpumpe (3) ein optischer Sensor (2) zur Blutgasanalyse längs des Schlauchleitungssystems angeordnet sind,
dass zwischen der ersten Fluidpumpe (3) und der therapeutisch wirksamen Komponente (5) ein zweiter Drucksensor (4) und zwischen der therapeutisch wirksamen Komponente (5) und der zweiten Fluidpumpe (8) ein dritter Drucksensor (6) längs des Schlauchleitungssystems angeordnet sind, und
dass zwischen der zweiten Fluidpumpe (8) und dem Auslass (A) ein Strömungsflusssensor (9) sowie ein Blasensensor längs des Schlauchleitungssystem angeordnet sind.

8. Anordnung nach einem der Ansprüche 1 bis 6,
**dadurch gekennzeichnet, dass** die wenigstens eine therapeutisch wirksame Komponente ein Oxygenator (5), vorzugsweise ein Membran-Oxygenator ist.

9. Anordnung nach den Ansprüchen 6 bis 8,
**dadurch gekennzeichnet, dass** sich der Kontrollalgorithmus durch wenigstens die nachfolgende Kontrollregel auszeichnet:
- Bei Unterschreiten des Sensorsignals des dritten Drucksensors unter einen vorgebbaren Schwellwert wird die zweite Fluidpumpe deaktiviert, bei Überschreiten des Schwellwertes aktiviert.

10. Anordnung nach einem der Ansprüche 1 bis 9,
**dadurch gekennzeichnet, dass** die erste Fluidpumpe (3) stromab des Einlasses (E) und die zweite Fluidpumpe (8) stromab zur ersten Fluidpumpe (3) längs des Schlauchleitungssystem angeordnet sind.

11. Anordnung nach einem der Ansprüche 1 bis 10,
**dadurch gekennzeichnet, dass** die erste Fluidpumpe (3) in einem Modus betreibbar ist, bei dem die erste Fluidpumpe (3) einen pulsatilen Fluidstrom innerhalb des Schlauchleitungssystems zu erzeugen vermag.

12. Anordnung nach einem der Ansprüche 5 bis 11,
**dadurch gekennzeichnet, dass** die Kontrolleinheit (11) den wenigstens einen Betriebsparameter der zweiten Fluidpumpe (8) derart einstellt, dass zwischen beiden Fluidpumpen (3, 8) innerhalb des Schlauchleitungssystems ein zur zweiten Fluidpumpe (8) hin abfallendes Strömungsdruckgefälle herrscht.
